# EUROPÄISCHE PATENTSCHRIFT

(11) **EP 0 957 359 B1**
(45) Veröffentlichungstag und Bekanntmachung des Hinweises auf die Patenterteilung: **15.12.2004**
(21) Anmeldenummer: 99109422.8
(22) Anmeldetag: 11.05.1999
(51) Int. Cl.: G01N 33/50, G01N 33/543, G01N 33/53, B01L 3/00

(54) **ELISPOT-Verfahren zur Quantifizierung der Aktivität von menschlichen und tierischen Zellen, zur insbesondere Blutzellen**
Quantification of blood cell activity with ELISPOT-assay
Quantification de l'activité de cellules sanguines utilisant une méthode ELISPOT

(30) Priorität: 13.05.1998 DE 19821289
(43) Veröffentlichungstag der Anmeldung: 17.11.1999
(73) Patentinhaber: Schöllhorn, Volkmar, Dr., 72479 Strassberg (DE); Lehmann, Paul Viktor, Prof. Dr., Cleveland Hts. Ohio, 44118 (US)
(72) Erfinder: Schöllhorn, Volkmar, Dr., 72479 Strassberg (DE); Lehmann, Paul Viktor, Prof. Dr., Cleveland Hts. Ohio, 44118 (US)
(74) Vertreter: Patentanwälte Ruff, Wilhelm, Beier, Dauster & Partner

(56) Entgegenhaltungen:
- WO-A-83/00047
- WO-A-90/04182
- US-A- 5 516 490

## Beschreibung

Die Erfindung betrifft ein Verfahren zur Bestimmung der Aktivität von menschlichen und tierischen Zellen, insbesondere Blutzellen, durch Inkubieren der Zellen in Gegenwart von eine Zellreaktion auslösenden Fremdstoffen nach der ELISA-Spot-Methode.

Zur Durchführung der Aktivitätsmessung von Zellen, anhand der ELISA-Spot-Methode, die auch ELIspot, genannt wird, werden normalerweise am Boden der Löcher einer Mikrotiterplatte Fangstoffe, insbesondere Antikörper oder Fangproteine, fixiert, die aus den zu untersuchenden Zellen abgegebene Stoffe binden können. In die Löcher der Mikrotierplatte, die üblicherweise eine 96-Lochplatte ist, werden bestimmte Mengen an Zellen enthaltenden Körperflüssigkeiten, insbesondere Blut, Blutserum oder Zellkulturen gegeben. Von den Zellen wird dabei im wesentlichen eine Monolayerschicht am Boden gebildet und zwar in unmittelbarer Nähe der Fangstoffe. Durch gleichzeitiges Vorhandensein von die Zellen chemisch oder immunologisch reizenden Fremdstoffen, insbesondere Antigenen, Allergenen, Viren oder anderen Zellen, die ihrerseits Stoffe abgeben, werden die Zellen der Körperflüssigkeit veranlaßt, spezifische Stoffe, insbesondere Abwehrstoffe oder Botenstoffe (Chemokine), abzugeben, die von den benachbarten Fangstoffen gebunden werden und zu einer die Aktivität einer Zelle widerspiegelnden Farbreaktion gebracht werden können.

Die WO 90/04182 beschreibt ein Verfahren zum Nachweis von Antikörpern oder Antigenen, die von individuellen Zellen abgegeben werden. Diese Substanzen werden mit Hilfe von weiteren markierten Antikörpern nachgewiesen, wobei die Nachweisreaktion anhand der Anzahl von Spots auf einem festen Substrat ausgewertet wird.

Bisher wurde bei der Aktivitätsmessung von Zellen die Anzahl der reaktiven Zellen gemessen, um daraus Rückschlüsse auf die Aktivität des Zellmaterials ziehen zu können. Erfindungsgemäß wurde erkannt, daß es nicht so sehr, bzw. nicht nur auf die Anzahl der reagierenden Zellen, sondern vor allem auch auf die Qualität der Reaktion der einzelnen Zellen ankommt. Einzelne Zellen reagieren auf den gleichen Reizstoff sehr unterschiedlich, was sich in der Quantität der abgegebenen Stoffe und damit in der Quantität der Farbreaktion jeder einzelnen Zelle ausdrückt. Erfindungsgemäß ist es deshalb vorgesehen, nicht nur die Anzahl der reaktiven Zellen zu ermitteln, sondern die Intensität der Gesamtreaktion. Diese erlaubt wesentlich weitergehende Schlüsse auf die Aktivität der Zellen.

Gegenstand der Erfindung ist somit ein Verfahren zur Bestimmung, insbesondere quantitativen Bestimmung der Aktivität von menschlichen und tierischen Zellen, insbesondere Blutzellen oder Zellkulturen, durch Inkubieren der Zellen in Gegenwart von eine Zellreaktion auslösenden Fremdstoffen nach der ELISA-Spot-Methode, wobei von den Zellen in Abhängigkeit ihrer Aktivität abgegebene spezifische Stoffe im örtlichen Bereich der jeweiligen Zellen aufgefangen und durch Farbreaktion als Dots sichtbar gemacht werden und wobei, bezogen auf eine bestimmte Grundfläche, die Gesamtfärbung gemessen und daraus die Gesamtaktivität der reaktiven Zellen bestimmt wird. Sichtbar bedeutet hier, mit optischen Methoden erkennbar, einschließlich Lumineszenz- und Floureszenzfärbungen.

Wenige, sehr aktive Zellen können bspw. eine genauso gute Immunabwehr besitzen, wie viele wenig reagierende Zellen. Deshalb erlaubt die erfindungsgemäße Methode wesentlich zuverlässigere und aussagekräftigere Auswertungen.

Gemäß einer bevorzugten Ausführungsform der Erfindung kann die Gesamtfärbung gemessen werden, indem die Anzahl der gefärbten Dots bzw. Spots und ihre (unterschiedliche) Größe erfaßt wird. Die Größe der Dots ist proportional zur Aktivität der einzelnen Zellen. Aus dem Produkt von Anzahl und Größe ergibt sich dann die Gesamtfärbung bzw. die Gesamtaktivität der Zellen.

Im übrigen können die bekannten Techniken und Auswertungen der ELISA-Spot-Methode angewendet werden. Es wird zweckmäßigerweise mit Mikrotiterplatten gearbeitet, an deren Lochboden die Fangstoffe fixiert sind, die die von den Zellen abgegebenen Stoffe spezifisch binden. Als vorbestimmte Grundfläche wird die Bodenfläche eines Lochs einer Mikrotiterplatte, insbesondere einer 96-Lochplatte verwendet.

Bei bekannten Aktivitätsmessungen nach der ELISA-Spot-Methode werden pro Loch der Mikrotiterplatte normalerweise 1 Mio. Zellen eingesetzt. Im Blut sind 1 Mio. Zellen gewöhnlich in einem Milliliter Blut enthalten, so daß für eine 96-Lochplatte ca. 100 Milliliter Blut erforderlich ist. Es wurde erkannt, daß so große Zellmengen gar nicht erforderlich sind, weil ein Reader eines automatischen Auswertungsgerätes die Reaktion einzelner Zellen erfassen kann und eine wesentlich geringere Zellzahl ausreicht, um statistisch gesicherte Ergebnisse erhalten zu können. Untersuchungen haben gezeigt, daß es nicht empfehlenswert ist, einfach weniger Blut in die Löcher zu geben, oder das Blut bzw. die sonstige Körperflüssigkeit entsprechend zu verdünnen. Die Erfindung geht bei einer weiteren bevorzugten Ausführungsform den Weg, die Grundfläche, d.h. insbesondere die Bodenfläche eines Lochs der Mikrotiterplatte im Vergleich zu den bekannten Grundflächen zu verkleinern. Erfindungsgemäß ist es somit möglich, mit einer wesentlich kleineren Menge an Körperflüssigkeit auszukommen und dabei die Zelldichte über der Grundfläche aufrechtzuerhalten.

Gemäß einer bevorzugten Ausführungsform der Erfindung ist es somit vorgesehen, daß eine Mikrotiterplatte mit gegenüber üblichen Mikrotiterplatten um mindestens die Hälfte verringerter Grundfläche verwendet wird, die Grundflächen der einzelnen Löcher insbesondere 15 - 50 Prozent der Grundflächen der Löcher üblicher Mikrotiterplatten entspricht. Dadurch kann das entsprechende Volumen an Körperflüssigkeit, insbesondere Blut oder Blutserum auf 15 - 50 Prozent verringert werden, d.h. es können mit der gleichen Menge an Körperflüssigkeit zwei- bis sechsmal soviel Untersuchungen durchgeführt werden, wie bisher. Vorzugsweise ist die Grundfläche auf ca. 25 Prozent verringert, so daß viermal soviel Versuche durchgeführt werden können bzw. nur ein Viertel des Volumens an Körperflüssigkeit benötigt wird.

Normale 96-Lochplatten haben einen Lochdurchmesser am Boden von 5 mm, was einer Grundfläche von etwa 20 mm² entspricht. Erfindungsgemäß ist es vorgesehen, mit Grundflächen zu arbeiten, die kleiner als 15 mm², insbesondere kleiner als 10 mm², sind. Die Mindestfläche dient dazu, den Zugang der für die üblichen 96-Lochplatten entwickelten Einrichtungen, insbesondere zum automatischen Füllen, Spülen, Entnehmen und Messen, verwenden zu können. Vorzugsweise beträgt die Grundfläche jeweils 3 - 8 mm². Auf die genauere Ausgestaltung der entsprechenden Mikrotiterplatte wird nachfolgend noch eingegangen.

Im Blut gibt es Millionen verschiedener Antikörper, Millionen verschiedener B-Zellen. In entsprechender Vielzahl liegen auch die T-Helferzellen vor. Sie erkennen bestimmte Antigene und scheiden aktivierende Proteine aus, die die B-Zellen beeinflussen. Es gibt zwei verschiedene Arten von Helferzellen. Die einen, die sogenannten TH-1-Zellen, aktivieren die Immunabwehr, z.B. die Aktivität der B-Zellen, die anderen, die TH-2-Zellen supprimieren die Immunabwehr, z.B. die Aktivität der B-Zellen. Normalerweise liegt zwischen den jeweiligen, spezifischen TH-1- und TH-2-Zellen im gesunden Körper ein Gleichgewichtszustand vor. Bei Allergien und Autoimmunkrankheiten ist dieses Gleichgewicht zugunsten der TH-1-Zell-Aktivität gestört. Deshalb zeigen Allergiker eine Überreaktion. Bei Tumoren ist das Gleichgewicht zugunsten der supprimierenden TH-2-Zell-Aktivität verschoben. Der Körper zeigt deshalb keine Abwehr gegen den Tumor.

Abweichungen von diesem Gleichgewicht erlauben wertvolle Rückschlüsse auf das Vorhandensein und das Ausmaß von Allergien und Krankheiten. Es ist deshalb bevorzugt, mehrere Parameter gleichzeitig messen zu können. Dies ist durch die Anwendung von Multicolorreaktionen möglich.

TH-1- und TH-2-Helferzellen scheiden unterschiedliche Lymphokine (Botenstoffe) aus. Zum Beispiel scheiden TH-1-Zellen γ-Interferon und TH-2-Zellen Interleukin-4 oder -5 aus. Cytotoxische T-Zellen (Tc-Zellen) geben i.d.R. neben verschiedenen Lymphokinen (Botenstoffen) auch Perforine und Granzymes (Gruppe von Serinproteasen) ab. Neuerdings zeigt sich jedoch, daß innerhalb der Tc-Zellen einerseits zwischen unmittelbar cytotoxisch wirksamen Tc-Zellen, welche die genannten Perforine und Granzymes bilden, und andererseits Tc-Zellen mit einer vorwiegend unterstützenden Funktion zu differenzieren ist, wobei die letzteren keine Perforine und Granzymes abgeben. Durch die Verwendung entsprechend spezifischer Fangstoffe, insbesondere polyklonale und/oder monoklonale Antikörper, können die verschiedenen sekretierten Stoffe mit dem erfindungsgemäßen Verfahren erfaßt werden. Über Sekundär-Reaktionen können unterschiedliche Farben erzeugt werden, so daß durch getrennte Erfassung der unterschiedlich gefärbten Dots eine Aussage über die Aktivität verschiedener Zellen möglich ist, und zwar gleichzeitig. Es können die verschiedenen Arten von Farbstoffen eingesetzt werden, wie sie bei der ELISA-Methode bekannt sind, so z.B. die Peroxidasereaktion oder Chemolumineszenz- und Fluoreszenz-Farbstoffe. Auf diese Weise ist es möglich, verschiedene, aus insbesondere verschiedenen Zellen freigesetzte, spezifische Stoffe von insbesondere verschiedenen Fangstoffen spezifisch zu binden und auf einer Grundfläche mindestens 2, vorzugsweise mindestens 3 verschiedene Farbreaktionen durchzuführen und die gebildeten Spots bzw. Dots nach Art der Farbe getrennt zu messen. Die Messung kann gleichzeitig oder nacheinander nach Farben getrennt erfolgen.

Die beim erfindungsgemäßen Verfahren erhaltenen Bilder bzw. Farbbilder werden insbesondere optisch vergrößert und in Bildpunkte zerlegt, die von einem Lesegerät getrennt erfaßt und ausgewertet werden. Die Bildpunkte können als Zeilen abgelesen werden. Ein Computer kann dann aus den erhaltenen, linearen Werten ein zweidimensionales Bild zusammensetzen. Auf diese Weise ist auch die getrennte Erfassung von gleichfarbigen, ineinanderfließenden Spots möglich. Das Multicolorverfahren, d.h. die Erzeugung von mehrfarbigen Bildern, erlaubt es unabhängig von Anzahl und Größe der Dots 7 bis 8 verschiedene Qualitäten von von den Zellen abgegebenen Stoffen (Botenstoffen) zu bestimmen und dies aus einer Aktivitätsreaktion in einem Loch bzw. einer Bodenfläche.

Die oben erwähnte Zelldichte ist normalerweise so gehalten, daß die Aktivitätsreaktionen der meisten Zellen als diskrete Dots auf dem Farbbild erscheinen. Liegen Zellen nahe beieinander oder zeigen sie eine große Aktivität, dann können die Dots ineinanderfließen. In einzelnen Fällen ist es möglich, daß die Reaktivität der Zellen so groß ist, daß im wesentlichen die gesamte Grundfläche mehr oder weniger vollständig gefärbt ist. Durch Messung der Gesamtfärbung, je nach Farbe, ist zwar eine Messung der Gesamtaktivität möglich. Eine Aufschlüsselung in Anzahl und Aktivität der jeweiligen Zellen nicht. Es wurde jedoch gefunden, daß es insbesondere im Mittelbereich der Grundfläche immer wieder einzelne Stellen gibt, an denen die Zelldichte etwas geringer ist und somit voneinander unterscheidbare Dots ermittelt werden können. Es ist deshalb bei einer Ausführungsform der Erfindung im Falle einer starken, im wesentlichen flächig ineinandergehenden Färbung der Dots vorgesehen, insbesondere im Bereich der Mitte der Färbung, Stellen zu ermitteln, an denen die Dots als einzelne Punkte identifizierbar sind, deren Größe zu messen und daraus die Anzahl der Dots auf der Gesamtfläche zu errechnen. Auf diese Weise ist es möglich, auch bei diesen besonderen Fällen, sämtliche gewünschten Daten zu erhalten.

Bei bekannten Verfahren nach der ELISA-Spot-Methode werden die Konzentrationen der Fang- und Detektionsstoffe in einem Bereich gehalten, in welchem eine höhere Konzentration von Fang- oder Detektionsstoffen zu keiner Verstärkung des Farbsignals führt. Bei den üblichen Verfahren befinden sich die Fang- oder Detektionsstoffkonzentrationen also in einem Sättigungsbereich. Es wurde erkannt, daß der Einsatz von Konzentrationen aus einem Bereich, in dem sich Konzentration und Farbsignal proportional zueinander verhalten, zu einem optimalen Kontrast zwischen großer und kleiner Stärke des Signals, insbesondere Fläche des Farbdots, führt. In einer bevorzugten Ausführungsform des erfindungsmäßigen Verfahrens werden demnach die Konzentrationen von Fang- und Detektionsstoffen aus dem im wesentlichen linearen Bereich der Beziehung von Konzentration und Signal gewählt.

Bei der Auswahl der Fang- und Detektionsstoffkonzentrationen kommt es nicht so sehr auf die absoluten Stoffkonzentrationen an; vielmehr ist die Affinität der Stoffe zu ihren jeweiligen Bindungspartnern entscheidend. Diese Affinitäten können sich abhängig von verschiedenen Faktoren, beispielsweise Lagerungszeit und Behandlung, bei den Fang- und Detektionsstoffen, insbesondere bei Antikörpern, verändern. Da die Konzentrationen bei dem erfindungsgemäßen Verfahren ein kritischer Punkt sein können, ist es daher wichtig, die wirklichen Affinitäten der Fang- und Detektionsstoffe mit Hilfe von Testlösungen zu bestimmen und davon abhängig die Auswahl der Konzentrationen zu treffen.

Das erfindungsgemäße Verfahren eignet sich für die vollautomatische Bildauswertung bzw. Vorauswertung. Hierzu ist es vorteilhaft, den Bildauswertungsgeräten aufgrund von Vorversuchen gefundene Erfahrungswerte einzugeben, um die Genauigkeit der Auswertung zu optimieren, insbesondere ein Rauschen zu unterdrücken. So kann in ein Auswertungsprogramm eingegeben werden, welche Art von Reaktion gemessen und interpretiert werden soll. Der Leser (Reader), bzw. das Auswertungsprogramm kann auf die jeweilige Biochemie und das damit verbundene Erscheinungsbild der Farbbilder eingestellt werden. Zu diesen Voreingaben gehören bspw. Empfindlichkeit, Kontrast, Mindestgröße der zu erfassenden Bildpunkte. Bei der Auswertung erfolgt als Mindestausgabe die Gesamtfärbung bezogen auf die Grundfläche. Bei mehrfarbigen Bildern erfolgt eine entsprechend getrennte Angabe je nach Farbe. Bei einfarbigen Bildern reicht eine Schwarz/Weiß-Wiedergabe aus. Zusätzlich kann das bspw. über Video oder eine CCD-Kamera erfaßte und in den Computer eingespeißte Bild ausgedruckt werden. Weitere bevorzugte Angaben sind die Anzahl der Zellen und die Größenverteilung der Dots, die der Aktivität der Zellen entspricht. Die getrennte Erfassung der Dots erfolgt zweckmäßigerweise in Durchmesser-Abstufungen von je 0,01 mm.

Das erfindungsgemäße Verfahren eignet sich aufgrund seiner verbesserten Aussagekraft in hervorragender Weise zur Diagnostik und auch zu einer darauf aufgebauten Immunisierung bzw. Therapie sowie deren Überwachung (Monitoring). Erfindungsgemäß ist es nicht nur möglich, das Vorhandensein einer bestimmten Krankheit durch Messung der immunologischen Aktivität der Zellen festzustellen, sondern auch das Stadium der Krankheit, insbesondere, ob eine akute Krankheit vorliegt oder die Krankheit bereits vergangen ist.

Dies ist beispielsweise durch unterschiedlich lange Inkubation der zu untersuchenden Zellen in Gegenwart der die Reaktion auslösenden Stoffe, wie Bakterien, Viren, Allergene und insbesondere Antigene möglich. Reagieren die Zellen schnell, d.h. innerhalb kurzer Inkubationszeit, bspw. im Bereich von 1 bis 5 Stunden, dann läßt dies auf das Vorliegen einer akuten Erkrankung schließen. Zeigt sich eine Reaktion durch Abgeben der Botenstoffe erst nach längerer Inkubationszeit von bspw. zwei bis drei Tagen, dann zeigt dies an, daß die Krankheit vergangen ist, aber das Gedächtnis der T-Helferzellen noch aktivierbar ist. Die Aussagen über das Stadium einer Erkrankung sind bei vielen Krankheiten für die Therapie von entscheidender Bedeutung. Bspw. kann bei Helicobacter pylori durch Serologie schlecht geprüft werden, ob die Infektion akut, chronisch oder vergangen ist. Das immunologische Gedächtnis sitzt in den T-Helferzellen und zeigt deshalb auch die geheilte Krankheit an. Bei einer akuten Erkrankung geben die T-Helferzellen bereits nach Inkubierung von zwei bis drei Stunden aufgrund sofortiger Reaktion entsprechende Botenstoffe ab und aktivieren B-Zellen, Makrophagen und andere immunkompetente Zellen. Zeigt sich bei einer Inkubationszeit von zwei bis drei Stunden keine Reaktion, jedoch in einer Parallelprobe, die zwei bis drei Tage inkubiert wurde, dann läßt sich daraus feststellen, daß derzeit keine akute Krankheit vorliegt, jedoch eine Krankheit vorlag, die überwunden ist. In ähnlicher Weise wertvoll ist diese Diagnostik für Toxoplasma-Erkrankungen, die besonders bei Schwangerschaften gefährlich sind. Auch hier kann durch Variation der Inkubationszeit festgestellt werden, ob die Erkrankung in akutem Stadium vorliegt, ob eine solche in früherer Zeit vorlag oder ob eine Krankheit besteht oder bestand. Das Erkennen des Stadiums einer Erkrankung durch Messung der Aktivität von Zellen in Abhängigkeit von der Inkubationszeit ist eine neue von der einzelnen Vorgehensweise unabhängige, diagnostische Qualität.

Außer den verbesserten Diagnosen sind erfindungsgemäß auch neue Ansätze zur Therapie möglich. So hat es sich gezeigt, daß es in vielen Fällen therapeutisch wirksam ist, wenn ein gestörtes Verhältnis von TH1-Helferzellen zu TH2-Helferzellen wieder in Ordnung gebracht wird, d.h. das normale Gleichgewicht wieder hergestellt wird.

### Autoimmunerkrankungen

### Rheuma

Bei Rheuma werden Collagenstrukturen, z.B. im Knie zerstört. Es gibt TH1-Zellen, die gegen angegriffenes Collagen reagieren und diese bekämpfen. In Folge anderer Erkrankungen, z.B. chronischer Infektionen, kann angegriffenes Collagen über längere Zeit im Körper vorhandensein. Die TH1-Zellen erkennen dies nach einiger Zeit als Feind, da es neben dem die Infektion auslösenden Virus als Fremdkörper verstanden wird. Die zunächst nur gegen Fremdkörper bzw. tote Materie gerichtete Aktivität der TH1-Zellen kann dann umgepolt werden und auch gegen ähnliche, lebende Materie, d.h. das körpereigene Collagen, gerichtet sein, wodurch sich das Verhältnis von TH1- zu TH2-Zellen zugunsten der TH1-Zellen, die in der Abwehr stimulierend wirken, verschiebt. Durch eine im Vergleich zu normalen Impfungen, die TH1-Zellen aktivieren, überdosierte Gabe an Antigen durch parenterale Verabreichung, kann erreicht werden, daß die TH2-Zellen vermehrt werden, die zu einer Suppression der Immunreaktion führen.

In ähnlicher Weise kann auch bei Allergien vorgegangen werden, indem die Allergene in so hoher Dosierung verabreicht werden, daß sie die Vermehrung von TH2-Zellen stimulieren, die dann in einem ausreichenden Maße supprimierende Botenstoffe abgeben. Auch bei Diabetes kann die Aktivität der entsprechenden TH1-, TH2- und TC-Zellen gegen GAD-Proteine oder andere Proteine, die bei Diabetes eine Rolle spielen, gemessen werden. Durch gezielte Gabe der antigenen Proteine kann das Gleichgewicht der aktiven Zellen wieder hergestellt werden, so daß die Produktion von Pankreasenzymen nicht mehr gestört ist.

### Infektionskrankheiten

### Mykobakterien

Manche Tuberkuloseinfektionen sind serologisch nicht nachweisbar. Bei einem Nachweis mit ELISA-Spot finden sich antigenspezifische T-Helferzellen. Eine Therapie kann über eine Immunisierung mit Antigen erfolgen, bis das Verhältnis von TH1- zu TH2-Zellen zugunsten der TH1-Zellen verschoben ist, damit diese eine ausreichende Aktivierung gegen die Bakterien auslösen. Eine blinde Immunisierung kann demgegenüber zu einer Supprimierung der Immunantwort führen oder eine Allergie hervorrufen, was gerade das Gegenteil bewirkt.

Helicobacter pylori ist bereits oben erwähnt worden. 60 bis 90 Prozent der Bevölkerung zeigen eine positive Reaktion. Aber nur ein bestimmter Prozentsatz bekommt eine Gastritis oder Magenkrebs. Untersuchungen haben gezeigt, daß diejenigen Patienten, die eine erhöhte antigenspezifische Reaktion von TH1-Zellen gegen Helicobacter zeigen, gefährdet sind. Infizierte Personen, bei denen das Gleichgewicht von TH1- zu TH2-Zellen stimmt, supprimieren eine Überempfindlichkeit gegenüber Helicobacter pylori und zeigen die Symptome nicht. Es zeigt sich deshalb eine Möglichkeit zur Therapie durch gezielte Gabe von Antigenen und anderen immunogenen Stoffen, um dadurch die antigenspezifischen TH1-Zellen zu reduzieren und die TH2-Zellen zu aktivieren.

### HIV

Bisher ist man davon ausgegangen, daß eine Verringerung der Population von T-Helferzellen bei HIV-Infektion am Ende zur AIDS-Symptomatik führt. Es wurde jedoch gefunden, daß nicht eigentlich die Zellzahl verringert ist, sondern die Aktivität bzw. Funktionalität dieser Zellen. Dies zeigt sich an einer stark verringerten Spot- bzw. Dot-Größe im Vergleich zur Normalgröße. Eine solche Aktivitätskontrolle bei HIV-infizierten Personen kann mittels ELIspot durchgeführt werden, indem als Fremdstoff bzw. die Reaktion auslösender Stoff ein Stoff verwendet wird, der im wesentlichen alle Aktivitäten der Helferzellen anspricht. Hier sind mitogen wirkende Stoffe, bspw. Tetanus geeignet. Aus der Erkenntnis, daß bei HIV-Infektion nicht die Zahl der T-Zellen, sondern deren Aktivität vermindert ist, bzw. wird, ergibt sich ein Ansatz zur Therapie bzw. zur Minderung der Folgen der Infektion.

Sobald festgestellt wird, daß die Spotgröße der T-Zellen des Patienten kleiner wird, muß versucht werden, durch eine Belebung der Aktivität der T-Zellen die beginnende oder bestehende Immunschwäche zu beseitigen. Es ist auch vorteilhaft, bei HIV infizierten Patienten die Immunabwehr dieser Patienten gegen die sogenannten Problemkeime, wie Toxoplasma, Zytomegalie-Virus, Mykobakterien, Lungenentzündung erzeugende Keime und dergleichen, an deren Infektionen die HIV-Patienten normalerweise sterben, zu prüfen, gegebenenfalls zu aktivieren und zu überwachen. Eine solche gezielte Behandlung ist wirkungsvoller und für den Patienten weniger belastend als ungezielte Breitbandtherapie.

### Krebs

Allgemein geht man davon aus, daß das Immunsystem keine starken Antworten gegen körpereigenen Krebs entwickeln kann. Es ist aber denkbar, daß sehr wohl im Körper eine Immunantwort vorhanden ist und krebsantigenspezifische T-Zellen produziert werden. Diese gehören jedoch dem TH2-Typ an und greifen den Tumor nicht an, da sie eher supprimierend wirken. Ein Therapieansatz besteht darin, durch gezielte Gabe von Krebsantigenen oder Immunmodulatoren die supprimierende Immunantwort in eine aktivierende Antwort umzuwandeln, d.h. aktivierende gegen den Krebs gerichtete TH1- und TC-Zellen zu generieren. Dies kann beispielsweise durch Verabreichung von Krebszellen und zugehörigem Adjuvans vorgenommen werden. Der Verlauf der Behandlung kann durch Überwachung der Zellaktivität verfolgt werden.

### Transplantationen

Es wurde festgestellt, daß die Menge und Stimulierbarkeit der TH2-Zellen ein Maß für die Nichtabstoßung ist. Je mehr TH2-Aktivität vorhanden ist, desto geringer ist das Abstoßungsrisiko. Durch Messungen der TH2-Aktivität bzw. des Gleichgewichts zwischen TH1- und TH2-Zellen im zeitlichen Zusammenhang mit der Transplantation kann deshalb festgestellt werden, wie groß das Abstoßungsrisiko ist. Besteht die Möglichkeit, vor der Transplantation das Verhältnis zugunsten der TH2-Aktivität zu verbessern, kann das Abstoßungsrisiko vermindert werden.

Die Erfindung bezieht sich vor allem auf die, insbesondere quantitative Aktivitätsbestimmung von Zellen und die daraus gezogenen bzw. ermöglichten Diagnosen. Entsprechende Therapien können durch gezielte stimulierende Immunisierung (Impfung) gegen bestimmte Erreger, gegebenenfalls in Verbindung mit stimulierenden Adjuvantien, vorgenommen werden, und zwar in den Fällen, in denen eine nicht ausreichende Immunabwehr gegen die Erreger vorliegt. Im Falle einer Überreaktion gegen Fremdstoffe bzw. Antigene, z.B. im Falle von Allergien oder Autoimmunkrankheiten, gehört zur Therapie eine stimulierende Behandlung von supprimierenden Zellen, gegebenenfalls in Verbindung mit supprimierenden Adjuvantien. Dabei gehört zur Therapie eine Überwachung der Immunaktivitäten während der Behandlungsdauer, um die Reaktion des Patienten auf die Behandlung feststellen zu können und die Dosis der zu verabreichenden Substanzen und die Dauer ihrer Verabreichung bzw. der Behandlung überprüfen und festlegen zu können.

Die Bestimmung der Zellaktivität gegen ganz bestimmte Fremdstoffe ermöglicht eine genaue Diagnose und gezielte Behandlung. Ein solches Vorgehen ist nicht nur aus Kostengründen sehr vorteilhaft im Vergleich zu unspezifischen Therapien. Die therapeutischen Erfolge sind besser, und es können gleichzeitig unerwünschte Nebenwirkungen vermieden werden.

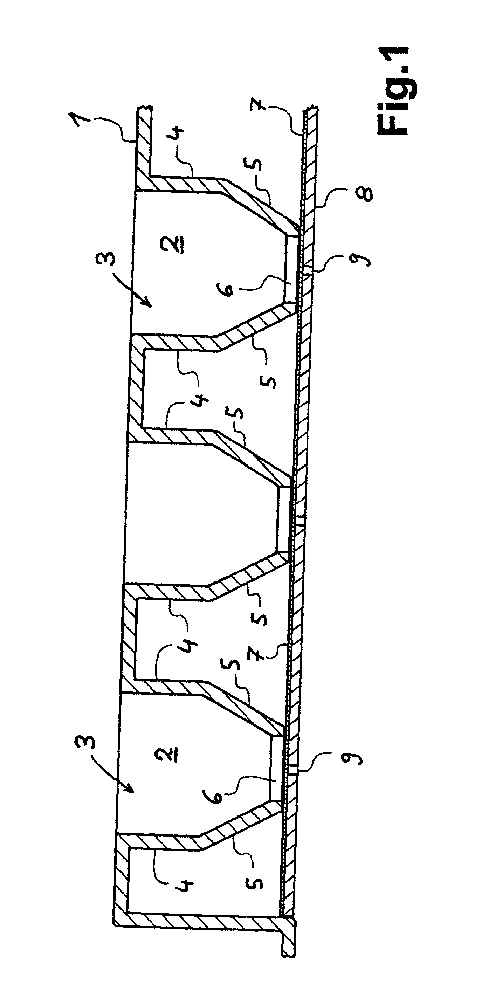

Weitere Merkmale der Erfindung,ergeben sich aus der nachfolgenden Beschreibung einer geeigneten Mikrotiterplatte in Verbindung mit der Zeichnung und den darauf gerichteten Ansprüchen.

Die Zeichnung zeigt einen Querschnitt durch eine Mikrotiterplatte nach der Erfindung.

Bekannte Mikrotiterplatten, die in der Regel als 96-Lochplatten ausgebildet sind, sind in der Regel massiv, wobei die Löcher als Sacklöcher ausgebildet sind, oder sie sind aus einer Folie tiefgezogen, so daß die Löcher an der Unterseite der Folie wie kleine Becher oder Näpfe vorstehen. Neuerdings ist man dazu übergegangen, den Boden der Sacklöcher porös auszugestalten, um bspw. Flüssigkeiten absaugen zu können und zu diesem Zweck werden die Lochplatten zunächst mit offenen Löchern hergestellt, die dann an der Unterseite mit porösen Membranen verschlossen werden. Zur mechanischen Sicherung der empfindlichen Membranen können auf diese von der Unterseite her dünne Abdeckplatten aufgelegt werden, die pro Loch eine Durchgangsöffnung aufweist. Die Löcher der Mikrotiterplatten haben normalerweise einen Durchmesser von ca. 5 mm und sind im wesentlichen zylindrisch ausgebildet. Zur besseren Ausformung bei der Herstellung kann eine geringe Konizität vorgesehen sein. Die Bodenfläche beträgt bei den herkömmlichen Mikrotiterplatten entsprechend dem Durchmesser von ca. 5 mm etwa 20 mm².

Die hier beschriebenen Mikrotiterplatten sind demgegenüber so ausgebildet, daß die Bodenfläche um mindestens 50 Prozent, vorzugsweise um ca. 75 Prozent vermindert ist. Trotzdem ist vorgesehen, daß die Mikrotiterplatten in der üblichen Weise eingesetzt werden können, d.h. in Verbindung mit den üblichen Pipetier-, Dosier-, Absaug- und Inkubiereinrichtungen. Bei der in der Zeichnung dargestellten Ausführungsform der Mikrotiterplatte 1 ist eine aus einer Polystyrol-Folie tiefgezogene Ausführung vorgesehen. Sie ist als 96-Lochplatte ausgebildet, wobei beispielhaft nur 3 Lochreihen angedeutet sind. Die Löcher 2 sehen im oberen Öffnungsbereich im wesentlichen gleich aus, wie bei den herkömmlichen Platten. Sie besitzen einen im wesentlichen zylindrischen Öffnungsquerschnitt mit einer kreisrunden Öffnung 3 mit einem Durchmesser von ca. 6 mm. An dem zylindrischen Abschnitt 4 schließt sich ein stark konischer Abschnitt 5 an, durch den der Lochquerschnitt auf ca. 25 Prozent des Eingangswertes verringert wird, d.h. auf ca. 5 mm². Am Lochboden 6 findet sich eine Öffnung entsprechenden Querschnitts, die durch Durchstanzen des Lochbodens ausgebildet ist. Sämtliche 96 Löcher der Mikrotiterplatte sind durch eine durchgehende, poröse Membran 7 abgedeckt, die auf die Lochränder aufgeklebt ist. Als Membran kann eine poröse Nylonmembran verwendet werden. Bevorzugt ist eine Membran der Firma Polyfiltronics, wie sie unter der Bezeichnung "Polyfiltronics PG-5" im Handel ist. Durch die poröse Membran können die Körperflüssigkeiten, Waschlösungen und dergleichen abgesaugt werden. Zum Schutz der empfindlichen Membran ist diese von der Unterseite her mit einer dünnwandigen Platte 8 bedeckt, die pro Loch der Mikrotiterplatte an entsprechender Stelle ein kleines Loch 9 besitzt.

Da beim ELISA-Spot-Verfahren die Zellansiedlung in Bodennähe erfolgt, d.h. in unmittelbarer Nähe der dort fixierten Fangstoffe, kann durch die Verwendung der beschriebenen Mikrotiterplatten eine starke Verringerung der benötigten Menge an Körperflüssigkeiten erzielt werden, im vorliegenden Fall wird die benötigte Menge auf ein Viertel verringert. Eine entsprechende Verringerung der Menge an Körperflüssigkeit kann auch bei anderen Ausgestaltungen der Mikrotiterplatte erreicht werden. So kann anstelle der abgestuften Konizität auch eine gleichmäßige Konizität von oben nach unten vorgesehen sein. Weiterhin kann der Bodenquerschnitt auch durch abgerundete Ausbildung der Unterseite des Loches, bspw. durch parabolische Formung der Löcher erreicht werden.

Der im folgenden beschriebene Elispot-Kit ist dazu geeignet, daß beanspruchte Verfahren besonders einfach durchzuführen. Der Kit umfaßt einen Träger, bevorzugt eine Mikrotiterplatte mit 96 Vertiefungen, einen oder mehrere auf diesem Träger fixierte Fangantikörper, ein oder mehrere Antigene, welche für die Aktivierung der vom Anwender bereitzustellenden Zellen geeignet sind, sowie mindestens einen weiteren Detektionsantikörper, welcher eine Markierung trägt. Diese Markierung ist derart gestaltet, daß eine optische Auswertung der Messung möglich ist. In einer geeigneten Ausführungsform des Elispot-Kits wird es sich dabei um ein mit dem Detektionsantikörper konjugiertes Enzym handeln, welches nach Zugabe entsprechender Reagenzien eine Farbreaktion katalysiert. Es sind jedoch auch Lumineszenz- , Fluoreszenz- oder Radioaktivmarkierungen möglich.

Ein wichtiges Merkmal des Elispot-Kits sind die eingestellten Konzentrationen der Antikörper. Die Konzentrationen sind aus dem linearen Bereich der Beziehung von Antikörperkonzentration und Signalstärke gewählt. Dies bewirkt einen optimalen Kontrast zwischen großer und kleiner Fläche des Signals, wodurch eine sehr aussagekräfige Auswertung der Meßergebnisse möglich ist.

Bei der Bereitstellung der Träger mit den fixierten Antikörpern wird diesem kritischen Punkt der Konzentrationen in der Weise Rechnung getragen, daß die jeweiligen Affinitäten der Antikörper zu ihren Bindungspartnern durch Verwendung von dem Fachmann geläufigen Methoden ermittelt werden und unter Berücksichtigung dieser Werte die optimalen Konzentrationen eingestellt werden. Zusätzlich werden hierbei die Affinitätsverluste berücksichtigt, die durch das Lyophilisieren und die Lagerung des Elispot-Kits entstehen.

Durch die Fixierung von zwei, drei oder mehr verschiedenen Fang- und Detektionsantikörpern in einem Elispot-Kit wird es ermöglicht, verschiedene von den Zellen abgegebene Stoffe parallel zu erfassen und auszuwerten, so daß differenzierte Aussagen zu den Aktivitäten der Zellen sowie Rückschlüsse auf die Zellpopulationen, denen die Zellen zuzuordnen sind, getroffen werden können.

Gegenüber herkömmlichen Verfahren zur Analyse von Zell-Aktivitäten stellt das erfindungsgemäße Verfahren eine wesentliche Verbesserung dar, denn durch die Anwendung dieses Verfahrens, insbesondere bei der Verwendung des beschriebenen Elispot-Kits, können ausgesprochen differenzierte Aussagen sowohl über die Gesamtaktivität als auch insbesondere über die Aktivität einzelner Zellen getroffen werden. Durch dieses Verfahren erschließen sich daher neue Möglichkeiten zur Feststellung und Beobachtung von antigenspezifischen Zellaktivitäten. So können beispielsweise Mangelzustände festgestellt oder im Verlauf einer Impfung die Reaktionen des Immunsystems differenziert beobachtet werden. Dies ermöglicht eine gezielte Steuerung der Impfung, indem die Dosierung des Impfstoffes auf die Reaktion des Immunsystems abgestimmt wird.

Die nun mögliche differenzierte Kontrolle der Zellen des Immunsystems erlaubt weiterhin einen neuen Therapieansatz, denn es wurde erkannt, daß durch die Behandlung des Immunsystems mit Fremdstoffen die antigenspezifischen Aktivitäten von Zellen beeinflußt werden können. Da diese Beeinflussung nun insbesondere durch den beschriebenen Kit sehr gut erfaßbar ist, kann auch ein Verfahren zur Veränderung der antigenspezifischen Aktivität von Zellen durch die Behandlung mit Fremdstoffen durchgeführt werden. Dieses Verfahren ist dadurch gekennzeichnet, daß durch die Verwendung der Fremdstoffe antigenspezifische Aktivitäten der Zellen in Form von sekretierten Stoffen aktiviert oder supprimiert werden. Als Fremdstoffe kommen insbesonders mitogen wirksame Stoffe, wie beispielsweise Tetanustoxin, in Frage. Weiterhin kann durch die Kombination von verschiedenen Fremdstoffen ein synergistischer Effekt auf die Aktivierung oder Supprimierung der antigenspezifischen Zellaktivitäten erzielt werden. Bei den Zellen, die durch das erfindungsgemäße Verfahren in ihrer antigenspezifischen Aktivität verändert werden, handelt es sich vorzugsweise um Zellen des Immunsystems. In bevorzugten Ausführungsformen sind dies T-Helferzellen, cytotoxische T-Zellen oder B-Zellen.

Weiter oben sind verschiedene Krankheitsfälle beschrieben, die zumindest teilweise auf falsche bzw. für den Krankheitsverlauf ungünstige Reaktionen bestimmter Zellen des Immunsystems zurückzuführen sind. Im Rahmen einer Therapie ist es durch das erfindungsmäßige Verfahren möglich, bestimmte Zellpopulationen von Körperflüssigkeiten, beispielsweise Blut, Lymphe oder Gelenkflüssigkeit, durch Verabreichung geeigneter Fremdstoffe gezielt in ihrer antigenspezifischen Aktivität zu beeinflussen. Beispielsweise im Fall einer Krebserkrankung wäre es dann sinnvoll, die krebsantigenspezifischen T-Zellen, die eher supprimierend wirken, durch gezielte und kontrollierte Behandlung mit Fremdstoffen in die Lage zu versetzen, gezielt cytotoxisch gegen die Tumorzellen vorgehen zu können. Prinzipiell kommen für einen solchen Therapieansatz alle Krankheiten, die mit einer Störung des Immunsystems einhergehen, bzw. die durch eine Aktivierung des Immunsystems positiv beeinflußt werden können, in Frage.

## Patentansprüche

1. Verfahren zur Bestimmung der Aktivität von menschlichen und tierischen Zellen, insbesondere Blutzellen, durch Inkubieren der Zellen in Gegenwart von eine Zellreaktion auslösenden Fremdstoffen nach der ELISA-Spot-Methode, wobei von den Zellen in Abhängigkeit ihrer Aktivität abgegebene, spezifische Stoffe im örtlichen Bereich der jeweiligen Zellen aufgefangen und durch Farbreaktion als Dots sichtbar gemacht werden, **dadurch gekennzeichnet, dass**, bezogen auf eine bestimmte Grundfläche, die Gesamtfärbung gemessen und daraus die Gesamtaktivität der reaktiven Zellen bestimmt wird.

2. Verfahren nach Anspruch 1, **dadurch gekennzeichnet, dass** bei der Messung der Gesamtfärbung die Anzahl der gefärbten Dots und deren unterschiedliche Größe erfasst werden.

3. Verfahren nach Anspruch 1 oder 2, **dadurch gekennzeichnet, dass** die vorbestimmte Grundfläche kleiner als 15 mm², insbesondere kleiner als 10 mm² ist.

4. Verfahren nach einem der vorhergehenden Ansprüche, **dadurch gekennzeichnet, dass** zur Durchführung der Bestimmung eine Mikrotiterplatte mit gegenüber üblichen Mikrotiterplatten um mindestens die Hälfte verringerten Grundflächen pro Loch verwendet wird, wobei die Grundflächen insbesondere 15 - 50 Prozent der Lochgrundflächen üblicher Mikrotiterplatten entsprechen.

5. Verfahren nach einem der vorhergehenden Ansprüche, **dadurch gekennzeichnet, dass** mit Volumina an Zellsubstanz, insbesondere Körperflüssigkeiten von ca. 40 Mikroliter bis 250 Mikroliter pro Grundfläche, insbesondere Loch, gearbeitet wird.

6. Verfahren nach einem der vorhergehenden Ansprüche, **dadurch gekennzeichnet, dass** verschiedene aus Zellen freigesetzte, spezifische Stoffe von insbesondere verschiedenen Fangstoffen spezifisch gebunden werden und auf einer Grundfläche mindestens zwei, vorzugsweise mindestens drei Farbreaktionen durchgeführt werden und die gefundenen Dots nach Art der Farbe getrennt erfasst und ausgewertet werden.

7. Verfahren nach einem der vorhergehenden Ansprüche, **dadurch gekennzeichnet, dass** die auf der Grundfläche erhaltenen Aktivitätsbilder vergrößert und in Bildpunkte zerlegt werden, die von einem Lesegerät erfasst und ausgewertet werden, wobei die Erfassung bei verschiedenfarbigen Bildern nach Farbe getrennt erfolgt.

8. Verfahren nach einem der vorhergehenden Ansprüche, **dadurch gekennzeichnet, dass** im Falle einer starken, im wesentlichen flächig ineinander überlaufenden Färbung der Dots, insbesondere im Bereich der Mitte der Färbung, Stellen ermittelt werden, an denen die Dots als einzelne Punkte identifizierbar sind, ihre Größe gemessen und daraus die Anzahl der Dots auf der Gesamtfläche ermittelt wird.

9. Verfahren nach einem der vorhergehenden Ansprüche, **dadurch gekennzeichnet, dass** die spezifischen Stoffe auffangende Fangstoffe in einem Konzentrationsbereich eingestellt sind, in welchem sich die Größe von Farbdots im wesentlichen linear zu der Fangstoffkonzentration verhält.

10. Verfahren nach einem der vorhergehenden Ansprüche, **dadurch gekennzeichnet, dass** die von den Fangstoffen gebundenen spezifischen Stoffe bindende Detektionsstoffe in einem Konzentrationsbereich verwendet werden, in dem sich die Größe von Farbdots im wesentlichen linear zu der Detektionsstoffkonzentration verhält.

11. Verfahren nach einem der vorhergehenden Ansprüche, **dadurch gekennzeichnet, dass** die Menge von Fang- und/oder Detektionsstoffen auf deren Affinität zum zu bindenden spezifischen Stoff bezogen ist.

12. Verfahren nach einem der vorhergehenden Ansprüche, **dadurch gekennzeichnet, dass** die Genauigkeit der Auswertung optimiert, insbesondere ein Rauschen unterdrückt wird, indem Bildauswertungsgeräten aufgrund von Vorversuchen gefundene Erfahrungswerte eingegeben werden.

13. Verfahren nach einem der vorhergehenden Ansprüche, **dadurch gekennzeichnet, dass** die verschiedene Größe der Dots und vorzugsweise auch ihre Anzahl in Abstufungen von 0,01 mm Dot-Durchmesser getrennt erfasst und ausgegeben werden.

14. Verfahren nach einem der vorhergehenden Ansprüche, **dadurch gekennzeichnet, dass** durch verschieden lange Inkubationszeit der zu untersuchenden Zellen in Gegenwart der Fremdstoffe festgestellt wird, ob eine akute oder eine vergangene Erkrankung vorliegt, vorzugsweise eine akute Erkrankung anhand einer kurzen Inkubationszeit von insbesondere einigen Stunden und eine abgeklungene Erkrankung anhand einer längeren Inkubationszeit von insbesondere einigen Tagen festgestellt wird.

15. Verfahren nach einem der vorhergehenden Ansprüchen, **dadurch gekennzeichnet, dass** insbesondere bei HIV-Patienten, die Gesamtaktivität von TH1- und TH2-Zellen und vorzugsweise auch von TC-Zellen bestimmt wird und eine therapeutische Aktivierung dieser Zellen des Patienten durch den Grad der Abweichung der Aktivität dieser Zellen des Patienten vom Normwert bestimmt wird, wobei vorzugsweise die Gesamtaktivität der Zellen durch Verwendung eines im wesentlichen alle Aktivitäten der zu untersuchenden Zellen ansprechenden Fremdstoffes ausgelöst und gemessen wird, wobei als Fremdstoff mitogen wirkende Stoffe bevorzugt sind.

16. Verfahren nach einem der vorhergehenden Ansprüche, **dadurch gekennzeichnet, dass** bei Patienten, insbesondere bei HIV-Patienten, die Aktivität von Zellen gegen Fremdstoffe, insbesondere Problemkeime, untersucht wird und eine therapeutische Aktivierung oder Supprimierung dieser Zellen durch den Grad der Abweichung der Aktivität dieser Zellen vom Normwert bestimmt wird.

## Claims

1. A method for determination of the activity of human and animal-derived cells, particularly blood cells, by incubating the cells in accordance with the ELISA spot technique in the presence of foreign agents inducing a cellular reaction, wherein specific substances which are released from the cells based on the cells' activity are captured in the local area of the respective cells and made visible as dots by a color reaction, **characterized in that** in relation to a certain ground area, the total staining is measured and used to determine the total activity of the reactive cells.

2. A method according to claim 1, **characterized in that** the measurement of the total staining comprises the number of the stained dots and their various sizes.

3. A method according to claim 1 or 2, **characterized in that** the pre-determined ground area is smaller than 15 mm², particularly smaller than 10 mm².

4. A method according to one of the previous claims, **characterized in that** for conducting the determination, a microtiter plate is used which has a ground area per well that is reduced by at least half compared to conventional microtiter plates, wherein the ground areas correspond specifically to 15 - 50 percent of the well ground areas of conventional microtiter plates.

5. A method according to one of the previous claims, **characterized in that** cell substance volumes, particularly body fluid volumes of approximately 40 microliters to 250 microliters per ground area, particularly per well, are used.

6. A method according to one of the previous claims, **characterized in that** various cell-released specific substances are bound specifically, particularly by various capturing agents, and on one ground area at least two, preferably at least three color reactions are conducted, and the dots found are captured and analyzed separately according to the color type.

7. A method according to one of the previous claims, **characterized in that** the activity images obtained on the ground area are enlarged and dissected into pixels which are captured and analyzed by a reader, the capture being performed separately according to the colors in images with different colors.

8. A method according to one of the previous claims, **characterized in that** in the case of a strong, essentially two-dimensional partial overlap of dot staining, particularly in the area of the center of the staining, patches are determined where the dots can be identified as single spots, their size can be measured and then used to determine the number of dots on the total area.

9. A method according to one of the previous claims, **characterized in that** the capturing agents capturing the specific substances are adjusted to a concentration range in which the size of staining dots is essentially linear in relation to the concentration of the capturing agent.

10. A method according to one of the previous claims, **characterized in that** the detection agents binding the specific substances bound by the capturing agents are used in a concentration range in which the size of staining dots is essentially linear in relation to the concentration of the detection agent.

11. A method according to one of the previous claims, **characterized in that** the amount of capturing and/or detection agents is related to their affinity to the specific substance to be bound.

12. A method according to one of the previous claims, **characterized in that** the specificity of the analysis is optimized, and particularly, that the background is suppressed, by entering known values that have been obtained in previous experiments into image analysis devices.

13. A method according to one of the previous claims, **characterized in that** the various sizes of the dots and preferentially also the number of dots is captured and recorded separately in increments of 0.01 mm dot diameter.

14. A method according to one of the previous claims, **characterized in that** by varying the incubation length of the cells to be studied in presence of the foreign agents it is determined if an acute or a past disease is present, where preferably an acute disease is detected by a short incubation period of especially several hours, and a past disease is detected by a longer incubation period of especially several days.

15. A method according to one of the previous claims, **characterized in that** especially in HIV patients the total activity of TH1 and TH2 cells and preferably also of TC cells is determined, and therapeutic activation of these patient cells is determined by the degree of deviation of the activity of these patient cells from the normal value, wherein preferably the total activity of the cells is induced and measured by using a foreign agent addressing essentially all activities of the cells to be studied, whereas substances acting as mitogens are preferred as foreign agents.

16. A method according to one of the previous claims, **characterized in that** in patients, especially in HIV patients, the activity of cells against foreign agents, especially pathogens, is studied, and therapeutic activation or suppression of these cells is determined by the degree of deviation of the activity of these cells from the normal value.

## Revendications

1. Procédé de détermination de l'activité de cellules humaines et animales, en particulier de cellules sanguines, par incubation des cellules en présence de substances étrangères déclenchant une réaction cellulaire selon la méthode ELISA par spots, dans lequel des substances spécifiques, délivrées par les cellules en fonction de leur activité, sont capturées, dans la zone desdites cellules et sont rendues visibles sous forme de points par réaction de couleur, **caractérisé en ce que** la coloration globale est mesurée sur une surface rapportée donnée et que cette mesure permet de déterminer l'activité globale des cellules réactives.

2. Procédé selon la revendication 1, **caractérisé en ce que**, lors de la mesure de la coloration globale, le nombre de points colorés et leur taille respective sont déterminés.

3. Procédé selon l'une des revendications 1 et 2, **caractérisé en ce que** la surface rapportée pré-déterminée est inférieure à 15 mm², en particulier inférieure à 10 mm².

4. Procédé selon une des revendications ci-dessus, **caractérisé par** l'utilisation, aux fins de détermination, d'une plaque microtiter dont les trous présentent une superficie au moins deux fois inférieure à celle des trous de plaques microtiter courantes, en particulier dont les trous présentent une superficie égale à 15 à 50 pour cent de celle de plaques microtiter courantes.

5. Procédé selon une des revendications ci-dessus, **caractérisé en ce que** le volume de substance cellulaire, en particulier des liquides corporels, sur lequel on travaille est de 40 microlitres environ à 250 microlitres environ par surface rapportée, en particulier par trou.

6. Procédé selon une des revendications ci-dessus, **caractérisé en ce que** diverses substances spécifiques libérées par les cellules sont liées spécifiquement par diverses substances capteuses en particulier et qu'au moins deux, de préférence au moins trois réactions de couleur sont effectuées par surface rapportée et que les points déterminés sont détectés et évalués séparément selon leur type et leur couleur.

7. Procédé selon une des revendications ci-dessus, **caractérisé en ce que** les traces d'activité obtenues sur la surface rapportée sont agrandies et translatées en points d'image qui sont ensuite détectés et évalués par un appareil de lecture et dont la détection s'effectue par couleur en cas d'images de différentes couleurs.

8. Procédé selon une des revendications ci-dessus, **caractérisé en ce qu'**en cas de forte coloration et, fondamentalement, de forte superposition spatiale des points, en particulier dans la zone médiane de la coloration, des endroits soient déterminés dans lesquels les points sont identifiables distinctement, leur taille est mesurée et, à partir d'elle, le nombre de points sur la surface totale est calculé.

9. Procédé selon une des revendications ci-dessus, **caractérisé en ce que** la gamme de concentrations des substances capteuses capturant les substances spécifiques est choisie de façon à ce que le rapport entre la taille des points colorés et la concentration en substances capteuses soit fondamentalement linéaire.

10. Procédé selon une des revendications ci-dessus, **caractérisé en ce que** des substances de détection liant les substances spécifiques liées par les substances capteuses sont employées dans une gamme de concentrations dans laquelle le rapport entre la taille des points colorés et la concentration en substances de détection est fondamentalement linéaire.

11. Procédé selon une des revendications ci-dessus, **caractérisé en ce que** la quantité de substances capteuses et/ou de détection est proportionnelle à leur affinité avec la substance spécifique à lier.

12. Procédé selon une des revendications ci-dessus, **caractérisé en ce que** l'exactitude de l'évaluation est optimisée, en particulier le bruit de fond supprimé, par l'introduction, dans les appareils d'évaluation d'images, de valeurs expérimentales récoltées lors d'essais préalables.

13. Procédé selon une des revendications ci-dessus, **caractérisé en ce que** les différentes tailles de point et leur nombre sont détectés et évalués distinctement par pas de 0,01 mm de diamètre de point.

14. Procédé selon une des revendications ci-dessus, **caractérisé en ce que** la variation du temps d'incubation des cellules à analyser en présence de substances étrangères permet de déterminer l'existence d'une maladie aiguë ou passée, de préférence une maladie aiguë à l'aide d'un temps d'incubation de peu de durée de quelques heures en particulier, et une maladie guérie à l'aide d'un temps d'incubation plus long, en particulier de quelques jours.

15. Procédé selon une des revendications ci-dessus, **caractérisé en ce que**, chez les patients VIH en particulier, l'activité globale des cellules TH1 et TH2 et, de préférence également des cellules TC, est déterminée et qu'une activation thérapeutique de ces cellules du patient peut être déterminée à l'aide du degré de déviation de l'activité de ces cellules du patient par rapport à la valeur normale, et, de préférence, que l'activité globale des cellules est déclenchée et mesurée par l'utilisation d'une substance étrangère réagissant fondamentalement à toutes les activités des cellules à analyser, de préférence une substance mitogène.

16. Procédé selon une des revendications ci-dessus, **caractérisé en ce que**, chez les patients, en particulier chez les patients VIH, l'activité des cellules vis-à-vis de substances étrangères, en particulier les germes pathogènes, est analysée et que l'activation ou la suppression thérapeutique de ces cellules est déterminé à partir du degré de déviation de leur activité par rapport à la valeur normale.
